# DEMANDE DE BREVET EUROPEEN

(11) **EP 1 396 486 A1**
(43) Date de publication de la demande: **10.03.2004**
(21) Numéro de dépôt: 03292177.7
(22) Date de dépôt: 04.09.2003
(51) Int. Cl.: C07D 207/14, C07D 207/09, C07D 521/00, C07D 487/08, C07D 207/08, C07D 207/16, A61K 7/13

(54) **Dérivés bis-paraphénylènediamine à groupement pyrrolidinyle et utilisation de ces dérivés pour la coloration de fibres kératiniques**

(30) Priorité: 09.09.2002 FR 0211133
(71) Demandeur: L'OREAL, 75008 Paris (FR)
(72) Inventeur: Sabelle, Stéphane, 75005 Paris (FR); Ramos, Laure, 92340 Bourg Lareine (FR); Leduc, Madeleine, Les Chèvrefeuilles, 75011 Paris (FR)
(74) Mandataire: Wattremez, Catherine

(57) **Abrégé**

L'invention a pour objet de nouveaux dérivés de bis-paraphénylènediamine à groupement pyrrolidinyle substitués par un radical cationique, les compositions tinctoriales les contenant ainsi que le procédé de teinture de fibres kératiniques à partir de ces compositions.

La présente invention permet en particulier d'obtenir une coloration des fibres kératiniques chromatique, puissante, peu sélective et tenace.

## Description

L'invention a pour objet de nouveaux dérivés bis-paraphénylènediamine à groupement pyrrolidinyle, les compositions tinctoriales les contenant ainsi que le procédé de teinture de fibres kératiniques à partir de ces compositions.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Dans le domaine de la coloration capillaire, la para-phénylènediamine, la paratoluène diamine sont des bases d'oxydation largement utilisées. Elles permettent d'obtenir avec des coupleurs d'oxydation des nuances variées.

Cependant, il existe un besoin de découvrir de nouvelles base d'oxydation présentant un meilleur profil toxicologique que la para-phénylènediamine et la paratoluène diamine, tout en permettant de conférer aux cheveux d'excellente propriétés d'intensité de couleur, de variété de nuances, d'uniformité de la couleur et de ténacité aux agents extérieurs

Il est déjà connu d'utiliser des dérivés de paraphénylènediamine subtitués par un groupement pyrrolidinique comme base d'oxydation pour la coloration de fibres kératiniques. Par exemple, le brevet US 5 851 237 décrit l'utilisation de dérivés 1-(4-aminophényl)pyrrolidine éventuellement substitués sur le noyau benzénique afin de remplacer la paraphénylènediamine.

Le brevet US 5 993 491 propose l'utilisation de dérivés de N-(4-aminophényl)-2-hydroxyméthylpyrrolidine éventuellement substitués sur le noyau benzénique et sur l'hétérocycle pyrrolidinique en position 4 par un radical hydroxy afin de remplacer la para-phénylènediamine.

Le brevet US 5 876 464 décrit des dérivés de paraphénylènediamine substitués par un groupement pyrrolidinique porteur en position 2 d'un radical carbamoyle.

La demande de brevet JP 11-158048 propose des compositions contenant au moins un composé choisi parmi des dérivés de 4-aminoaniline éventuellement substitués sur le noyau benzénique et dont un des atomes d'azote est compris dans un cycle de 5 à 7 chaînons carbonés.

Le document WO 02/45675 décrit des dérivés de paraphénylènediamines substitués par un radical pyrrolidinique porteur d'un groupe ammonium quaternaire particulier.

Il est clairement établi que ces composés ne permettent pas de conférer aux cheveux une coloration de qualité équivalente à celle obtenue avec la para-phénylènediamine ou avec la para-toluènediamine du fait d'un manque d'intensité et d'uniformité de la couleur.

Il existe donc un réel besoin de découvrir de nouvelles bases d'oxydation présentant à la fois un bon profil toxicologique et des propriétés telles que les compositions les contenant permettent de conférer aux cheveux d'excellentes propriétés d'intensité de couleur, de variété de nuances, d'uniformité de la couleur et de ténacité vis à vis des différentes agressions extérieures que peuvent subir les cheveux.

Le but de la présente invention est de développer de nouvelles compositions tinctoriales ne présentant pas les inconvénients des bases d'oxydation de la technique antérieure en fournissant de nouvelles compositions tinctoriales pour la teinture de fibres kératiniques qui sont capables d'engendrer des colorations intenses dans des nuances variées, peu sélectives particulièrement résistantes et qui présentent un bon profil toxicologique.

Ce but est atteint avec la présente invention qui a pour objet des dérivés de bis-para-phénylènediamine substitués par un groupement pyrrolidinyle de formule (I) et leurs sels d'addition 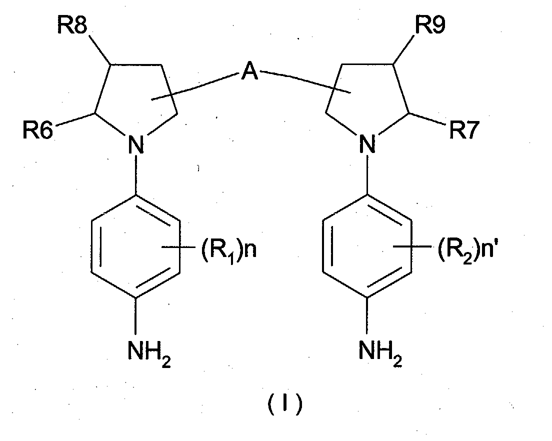 dans laquelle :
- n et n', indépendamment l'un de l'autre sont compris entre 0 et 4 inclus, étant entendu que lorsque n, respectivement n' est supérieur ou égal à 2 alors les radicaux R₁ et R₂ peuvent être identiques ou différents,
- R₁ et R₂ représentent indépendamment un atome d'halogène ; une chaîne hydrocarbonée en C₁-C₆, aliphatique ou alicyclique, saturée ou insaturée, un ou plusieurs des atomes de carbone de la chaîne pouvant être remplacés par un atome d'oxygène, d'azote, de silicium, de soufre, par un groupement SO ou par un groupement SO₂, les radicaux R₁ et R₂ ne comportant pas de liaison peroxyde, ni de radicaux diazo, nitro ou nitroso , la chaîne pouvant être substituée par un ou plusieurs atomes d'halogène, un ou plusieurs radicaux hydroxy, alcoxy en C₁-C₆, amino, mono- ou di- alkyl(C₁-C₆)amino, trialkyl(C₁-C₆)ammonium, un radical N-alkyl(C₁-C₆)imidazolinium
- A est une liaison covalente ou une chaîne alkylène comportant de 1 à 14 atomes de carbone, linéaire ou ramifiée, saturée ou insaturée, un ou plusieurs des atomes de carbone de la chaîne pouvant être remplacés par un radical onium Z et/ou par un atome choisi parmi les atomes d'oxygène, de soufre, de silicium ou d'azote, un groupe CO, SO ou SO₂, la chaîne pouvant être substituée par un ou plusieurs atomes d'halogène, un ou plusieurs radicaux hydroxyle, alcoxy en C₁-C₆, amino, alkyl(C₁-C₆)amino ou di-alkyl(C₁-C₆)amino ;
- R₆ et R₇ représentent indépendamment un atome d'hydrogène ; un radical carboxyle ; un radical alkyl(C₁-C₄)carboxyle ; un radical carbamoyle ; un radical (alkyl ou dialkyl)(C₁-C₄) carbamoyle ; un radical trialkyl(C₁-C₆)silane, un radical tri(alkyl(C₁-C₆)ammonium ; un radical N-alkyl(C₁-C₆)imidazolinium ; un radical alkyle en C₁-C₁₅ ; un radical alkyle en C₁-C₁₅pouvant être insaturé, substitué par un ou plusieurs radicaux hydroxy, alkyl(C₁-C₆)oxy, amino, mono- ou di-alkyl(C₁-C₆)amino, thiol, alkyl(C₁-C₆)sulfonique ou halogène ; un radical alkyle en C₁-C₁₅ pouvant être insaturé, substitué par un ou plusieurs radicaux carboxylique, alkyl (C₁-C₆)carbonyle, alcoxy (C₁-C₆)carbonyle, carbamoyle, mono- ou di-alkyl (C₁-C₆)carbamoyle, trialkyl(C₁-C₆)silane, tri(alkyl(C₁-C₆)ammonium ou N-alkyl(C₁-C₆)imidazolinium ;
- R₈ et R₉ représentent indépendamment un atome d'hydrogène, un radical hydroxyle ; un radical alkyl(C₁-C₄)oxy, un radical amino ; un radical mono- ou di-alkyl(C₁-C₄)amino ; un radical thiol ; un radical carboxyle ; un radical alkyl(C₁-C₄)carboxyle ; un radical carbamoyle ; un radical (alkyl ou dialkyl)(C₁-C₄) carbamoyle ; un radical trialkyl(C₁-C₆)silane, un radical tri(alkyl(C₁-C₆)ammonium ; un radical N-alkyl(C₁-C₆)imidazolinium ; un radical alkyle en C₁-C₁₅ ; un radical alkyle en C₁-C₁₅ pouvant être insaturé, substitué par un ou plusieurs radicaux hydroxy, alkyl(C₁-C₆)oxy, amino, mono- ou di-alkyl(C₁-C₆)amino, thiol, alkyl(C₁-C₆)sulfonique ou halogène ; un radical alkyle en C₁-C₁₅ pouvant être insaturé, substitué par un ou plusieurs radicaux carboxylique, alkyl (C₁-C₆)carbonyle, alcoxy (C₁-C₆)carbonyle, carbamoyle, mono- ou di-alkyl (C₁-C₆)carbamoyle , trialkyl(C₁-C₆)silane, tri(alkyl(C₁-C₆)ammonium ou N-alkyl(C₁-C₆)imidazolinium.

L'invention a aussi pour objet une composition tinctoriale comprenant au moins un dérivé de bis-para-phénylènediamine de formule (I) à titre de base d'oxydation.

Un autre objet de l'invention est l'utilisation de cette composition pour la teinture de fibres kératiniques et le procédé de teinture de fibres kératiniques, en particuliers les fibres kératiniques humaines telles que les cheveux mettant en oeuvre la composition de la présente invention.

La composition de la présente invention permet en particulier d'obtenir une coloration de fibres kératiniques chromatique, puissante, peu sélective et tenace.

Dans le cadre de l'invention, une chaîne hydrocarbonée aliphatique est une chaîne linéaire ou ramifiée pouvant contenir des insaturations du type alcène ou alcyne. Une chaîne hydrocarbonée alicyclique est une chaîne ramifiée saturée ou insaturée ne contenant pas de structure cyclique aromatique.

Lorsque la chaîne est interrompue par un atome T d'oxygène, de soufre, d'azote, de silicium ou SO₂, on obtient par exemple un motif CH₂-T-CH₂, -T-; etc.

A titre d'exemple, R₁ et R₂ indépendamment, peuvent être un atome de chlore, de brome, un radical méthyle, éthyle, isopropyle, vinyle, allyle, méthoxyméthyle, hydroxyéthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxyéthyle, 1,2- diaminoéthyle, méthoxy, éthoxy, allyloxy, 2-hydroxyéthyloxy.

Selon un mode de réalisation particulier, R₁ et R₂ sont indépendamment choisis parmi le chlore, le brome, un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄, un radical trialkyl(C₁-C₄)ammonium-alkyle en C₁-C₄, un radical N-alkyl(C1-C4)imidazoliniumalkyle en C₁-C₄. Par exemple, R₁ et R₂ sont choisis parmi un radical méthyle, isopropyle, terbutyle, hydroxyméthyle, 2-hydroxyéthyle, 1,2-dihydroxyéthyle, méthoxy, isopropyloxy, 2-hydroxyéthoxy, triméthylammoniumméthyle, N-méthylimidazolinium.

Dans la formule (I), n et n' sont indépendamment 0 ou 1.

Selon un mode de réalisation particulier, A ne comporte pas de liaison peroxyde, ni de radicaux diazo, nitro ou nitroso.

A est de préférence une liaison covalente ou une chaîne alkylène comportant de 1 à 8 atomes de carbone. Selon un mode de réalisation particulier, A est une chaîne alkylène comprenant de 1 à 8 atomes de carbone, un ou plusieurs des atomes de carbone de la chaîne pouvant être remplacés par un atome d'azote et/ou un atome d'oxygène. A peut représenter une chaîne alkylène, dont un ou plusieurs atomes de carbone de la chaîne peuvent être remplacés par un atome d'azote et/ou d'oxygène, et comportant un ou plusieurs radicaux onium Z.

On entend par le terme "onium" un radical quaternaire d'une base azotée.

Selon un premier mode de réalisation, le radical onium Z correspond à la formule (II) : 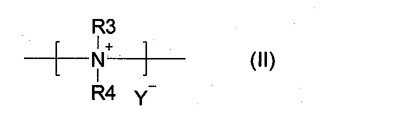 dans laquelle
- Y⁻ est un contre ion
- R₃ et R₄, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, amido ou alkyl(C₁-C₆)sulfonyle ;
- R₃ et R₄ ensemble, forment, avec l'atome d'azote auxquels ils sont rattachés, un hétérocycle à 5, 6 ou 7 chaînons ;
- lorsqu'au moins 2 radicaux oniums de formule (II) sont présents dans la chaîne A, les radicaux R₃ et/ou R₄ d'un des radicaux onium peuvent former avec les radicaux R₃ et/ou R₄ de l'autre radical onium une structure cyclique diammonium.

Dans ce dernier cas, les structures qui sont obtenues en reliant deux groupes R₃ ou R₄ de deux radicaux oniums présents dans la chaîne A sont par exemple :

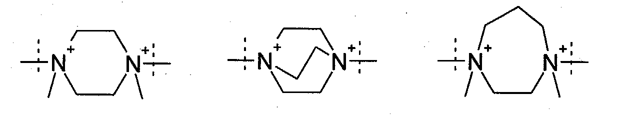

R₃ et R₄, pris séparément sont par exemple choisis parmi les radicaux alkyles en C₁-C₄, les radicaux hydroxyalkyles en C₁-C₄. Ils peuvent aussi former ensemble un cycle cationique pyrrolidinium pipéridinium, pipérazinium ou morpholinium, le cycle cationique pouvant être substitué par un atome d'halogène, un radical hydroxyle, alkyle en C₁-C₆, monohydroxyalkyle en C₁-C₆, polyhydroxyalkyle en C₂-C₆, alcoxy en C₁-C₆, trialkyl(C₁-C₆)silanealkyle en C₁-C₆, amido, carboxyle, alkyle(C₁-C₆), thio, thioalkyle en C₁-C₆, alkyl(C₁-C₆)thio, amino, amino mono ou disubstitué par un radical alkyl(C₁-C₆), alkyl(C₁-C₆)carbonyle, amido ou alkyl(C₁-C₆)sulfonyle. A titre d'exemple, R₃ et R₄ ensemble forment un cycle cationique pyrrolidinium, pipéridinium et morpholinium.

Le radical onium Z peut aussi être un radical de formule (III) : 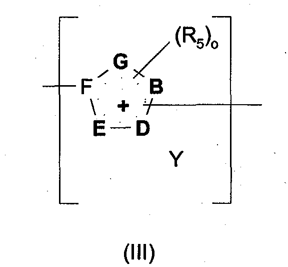 dans laquelle
- les sommets B, D, E, F, G identiques ou différents, représentent un atome de carbone, d'oxygène, de soufre ou d'azote necessaire pour former un cycle aromatique cationisé sur l'azote de type pyrolium, pyrazolium, imidazolium, triazolium, oxazolium, isooxazolium, thiazolium, isothiazolium ;o est un nombre entier compris entre 0 et 4 inclus ;
- R₅, identique ou différent, représente un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; lorsque les radicaux R₅ sont portés par un carbone, ils représentent également un radical hydroxyle, alkyl(C₁-C₄)oxy, amino, alkyl(C₁-C₄)amino ou di alkyl(C₁-C₄)amino, et
- Y est un contre-ion.

Dans ce cas, Z est de préférence choisi parmi le cycle imidazolium et le cycle thiazolium.

Le radical onium Z peut encore être un radical de formule (IV) : 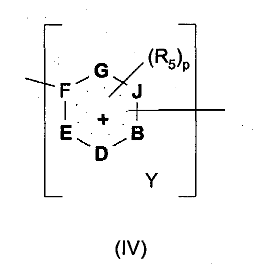 dans laquelle :
- les sommets B, D, E, F, G et J, identiques ou différents, représentent un atome de carbone ou d'azote pour former un cycle aromatique cationisé sur l'azote de type pyridinium, pyrimidinium, pyrazinium, triazinium et pyridazinium ;
- p est un nombre entier compris entre 0 et 4 inclus ;
- R₅, identique ou différent, représente un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; lorsque les radicaux R₅ sont portés par un carbone, ils représentent également un radical hydroxyle, alkyl(C₁-C₄)oxy, alkyl(C₁-C₄)amino ou di alkyl(C₁-C₄)amino, et
- Y représente un contre ion.

Dans ce cas, le radical onium Z de formule (IV) est de préférence un cycle cationisé pyridinium.

Dans le cadre de la présente invention, R₅ est de préférence un radical alkyle en C₁-C₄ ou hydroxyalkyle en C₁-C₄.

R₆ et R₇ peuvent être choisis de préférence parmi l'hydrogène, un radical hydroxyalkyle en C₁-C₄, par exemple hydroxyméthyle, un radical alkyle en C₁-C₄ par exemple méthyle, un radical carboxyle, un radical carbamoyle, un radical mono ou dialkyl(C₁-C₄)) carbamoyle par exemple N,N diméthylcarbamoyle, un radical trialkyl(C₁-C₄)ammoniumalkyl en C₁-C₄ par exemple triméthylammoniumméthyle, un radical N-alkyl(C₁-C₄)imidazoliumalkyle en C₁-C₄.

R₈ et R₉ sont choisis parmi l'hydrogène, un radical hydroxyle, un radical amino, un radical mono ou dialkyl (C₁-C₄))amino par exemple diméthylamino et di-2-hydroxyéthylamino, un radical alkyle en C₁-C₄ par exemple méthyle, un radical trialkyl(C₁-C₄)ammonium par exemple triméthylammonium, un radical N-alkyl(C₁-C4)imidazolinium par exemple N-méthyle imidazolinium.

Selon un mode de réalisation particulier, le dérivés de l'invention a la formule (I') suivante 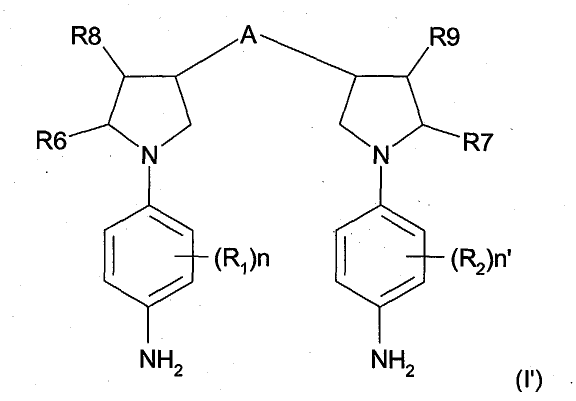 dans lesquels R₁, R₂, n, n', R₆, R₇, R₈, R₉ et A sont tels que définis précédemment.

De préférence dans la formule (I'), n et n' sont 0 ou 1, R₁ et R₂ sont choisis parmi un radical méthyle, isopropyle, terbutyle, hydroxyméthyle, 2-hydroxyéthyle, 1,2-dihydroxyéthyle, méthoxy, isopropoxy, 2-hydroxyéthoxy, triméthylammoniumméthyle, un radical N-méthylmidazoliniumméthyle ,R₆ et R₇ sont choisis parmi un radical hydrogène, hydroxyalkyle en C₁-C₄ par exemple hydroxyméthyle, un radical alkyle en C₁-C₄ par exemple méthyle, un radical carboxyle, un radical carbamoyle, un radical mono ou dialkyl(C₁-C₄)carbamoyle par exemple N,N diméthylcarbamoyle, un radical trialkyl(C₁-C₄)ammoniumalkyle en C₁-C₄ par exemple triméthylammoniumméthyle, un radical N-alkyl(C₁-C₄)imidazoliumalkyle, R₈ et R₉ sont choisis parmi un atome d'hydrogène, un radical hydroxyle, un radical amino, un radical mono ou dialkyl (C₁-C₄))amino par exemple diméthylamino et di-2-hydroxyéthylamino, un radical alkyle en C₁-C₄ par exemple méthyle, un radical trialkyl(C₁-C₄)ammonium par exemple triméthylammonium, un radical N-alkyl(C₁-C₄)imidazolinium par exemple N-méthyle imidazolinium, A est une liaison covalente ou un radical choisi parmi :

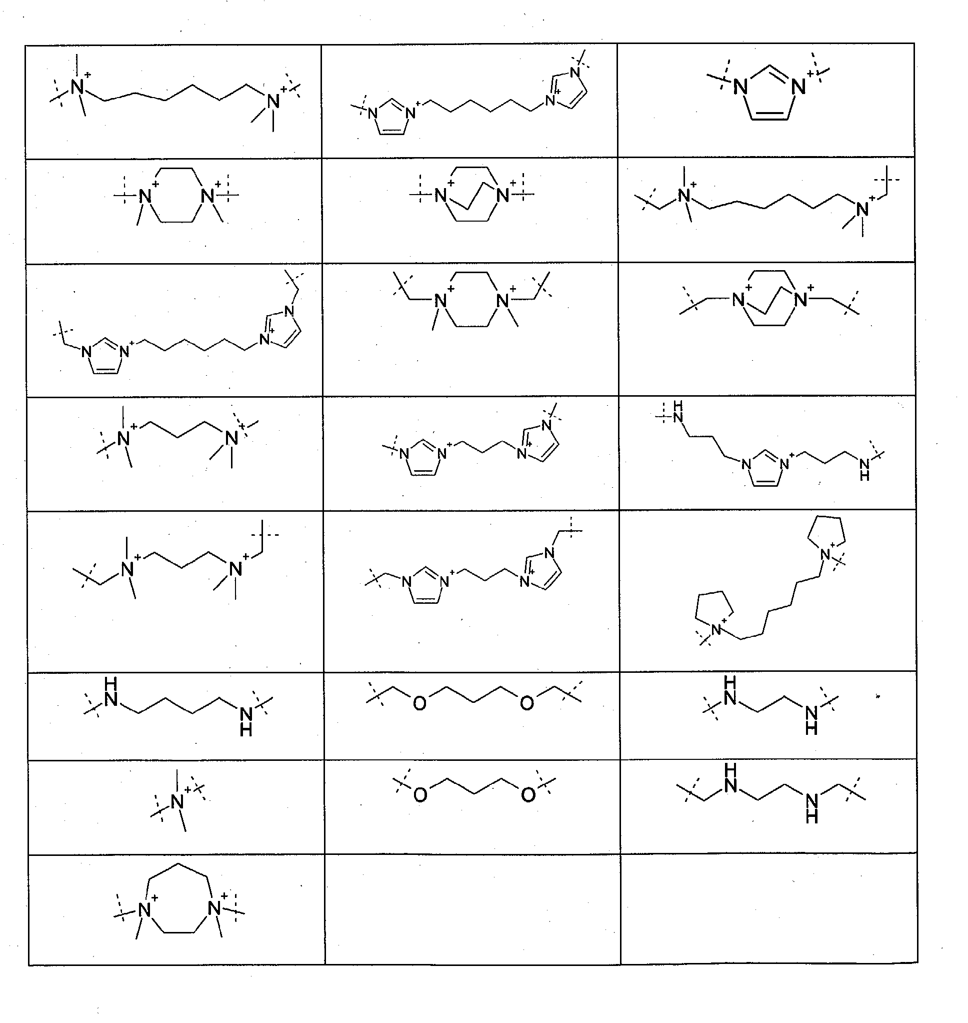

Dans le cadre de l'invention, le contre ion Y peut être choisi parmi un atome d'halogène tel que le brome, le chlore, le fluor ou l'iode, un hydroxyde, un citrate, un succinate, un tartrate, un lactate, un tosylate, un mésylate, un benzènesulfonate, un acétate, un hydrogènesulfate ou un alkylsulfate en C₁-C₆ tel que par exemple le méthylsulfate, ou l'éthylsulfate.

A titre d'exemples de dérivés de formule (I), on peut citer :

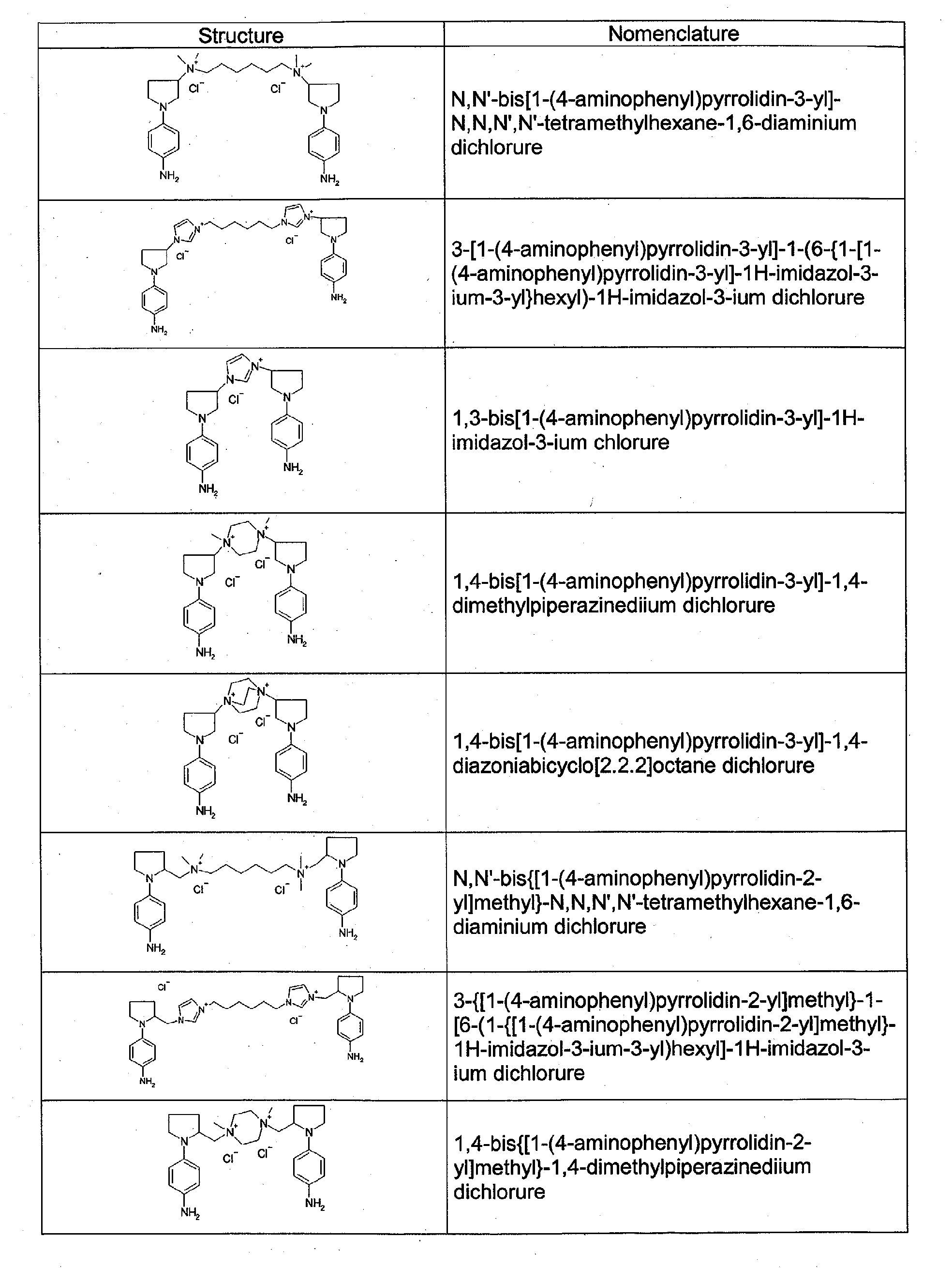

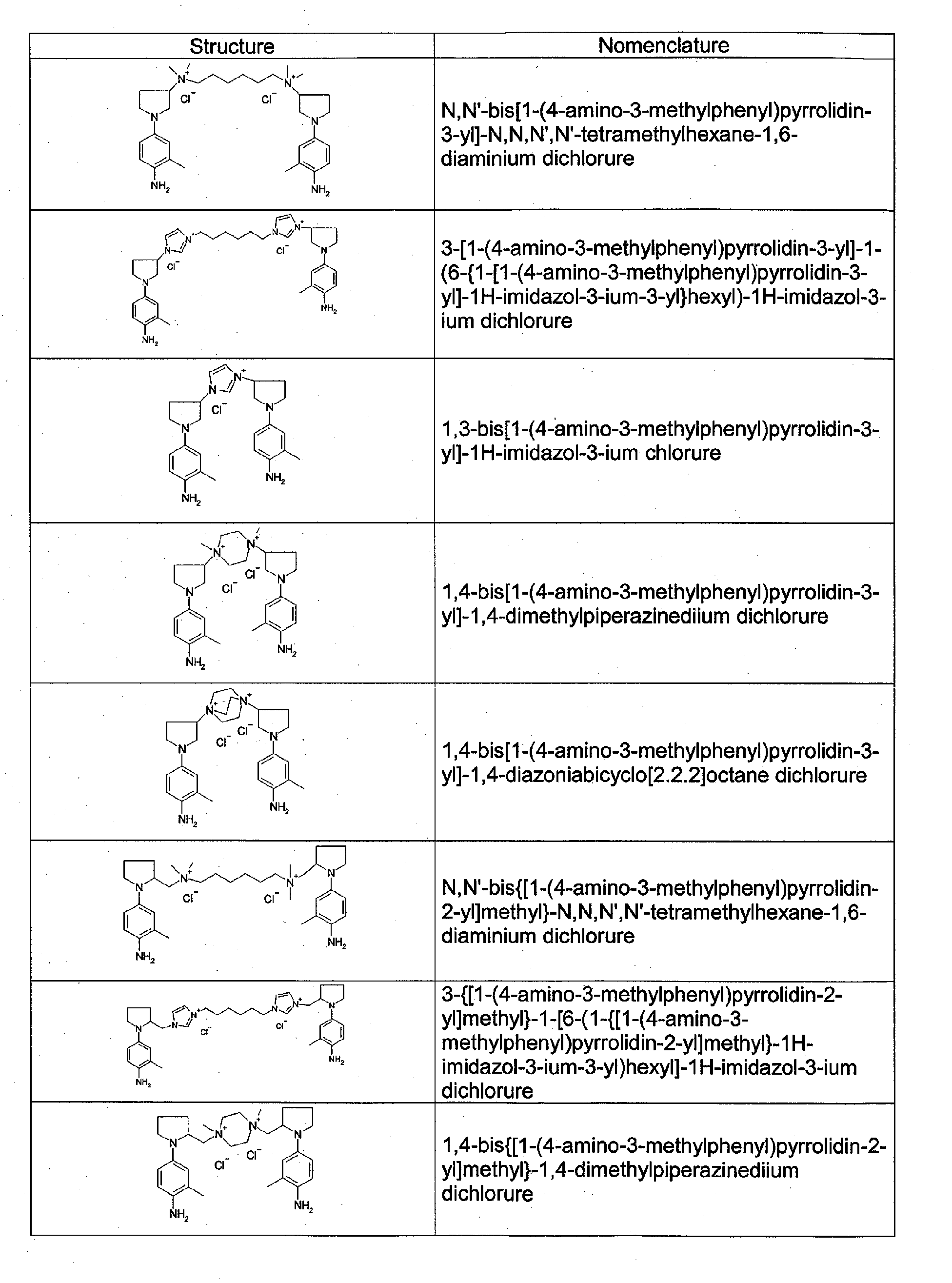

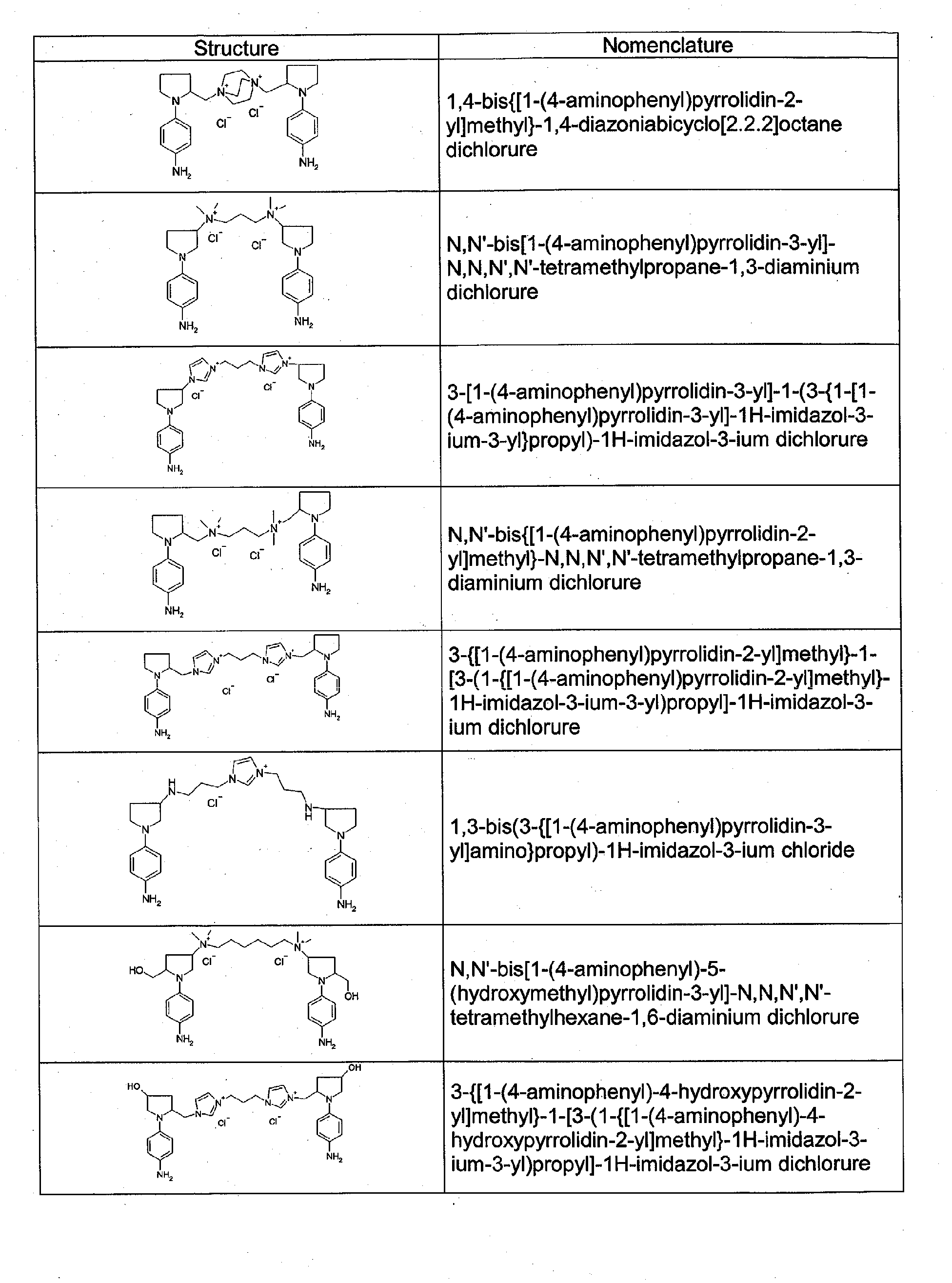

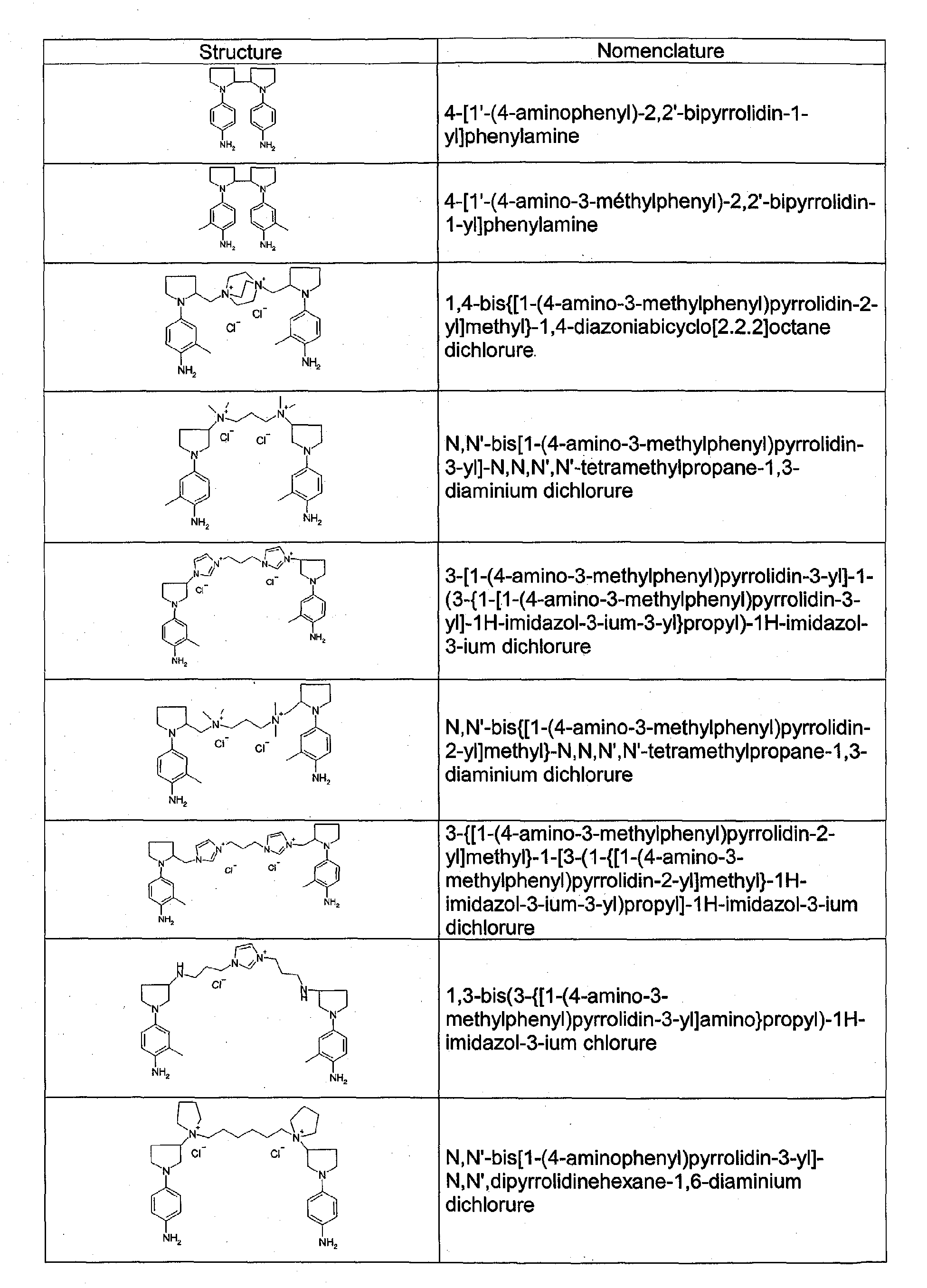

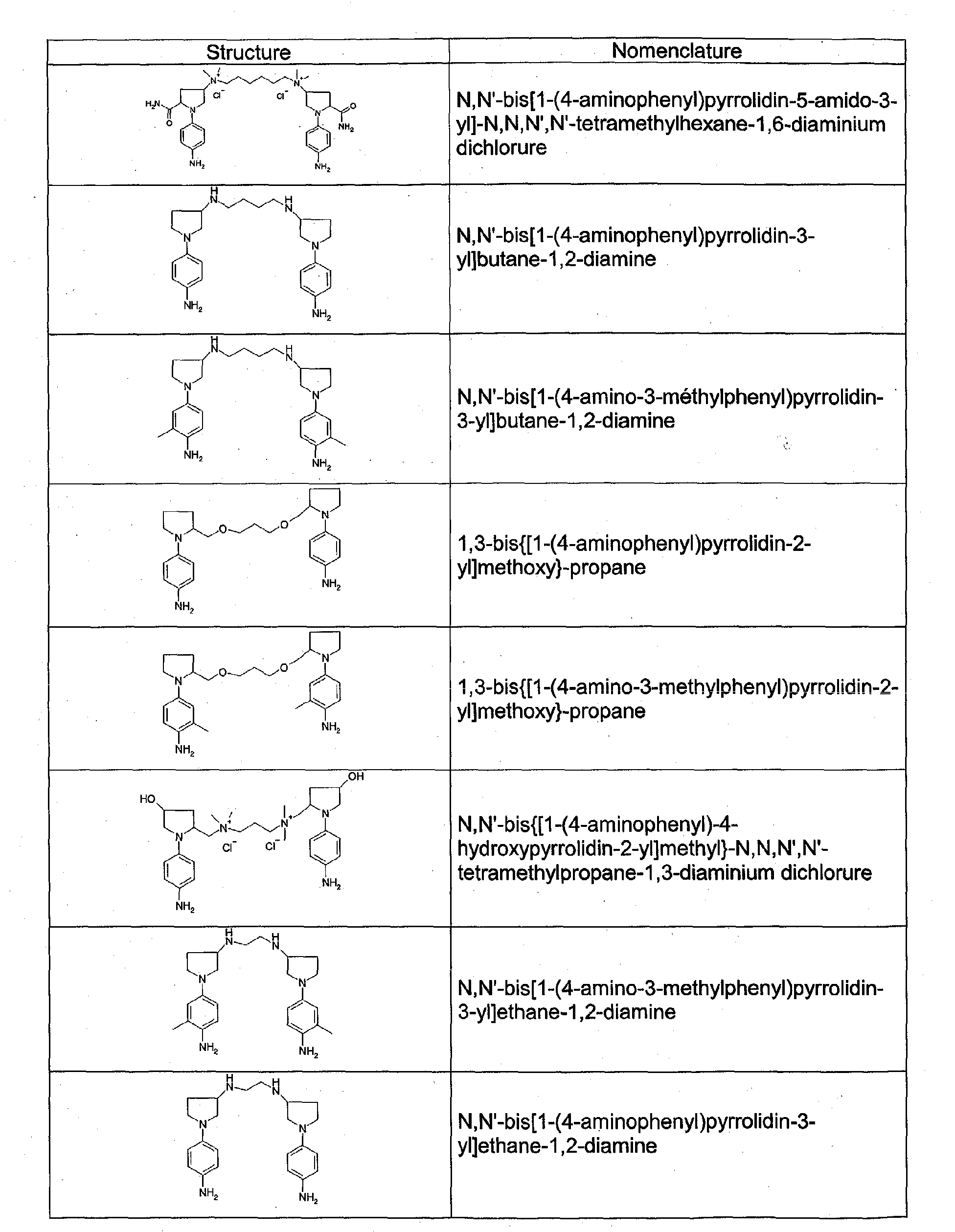

De préférence, les composés de formule (I) sont choisis parmi
- N,N'-bis[1-(4-aminophenyl)pyrrolidin-3-yl]-N,N,N',N'-tetramethylhexane-1,6-diaminium dichlorure
- 3-[1-(4-aminophenyl)pyrrolidin-3-yl]-1-(6-{1-[1-(4-aminophenyl)pyrrolidin-3-yl]-1H-imidazol-3-ium-3-yl}hexyl)-1H-imidazol-3-ium dichlorure
- 1,3-bis[1-(4-aminophenyl)pyrrolidin-3-yl]-1H-imidazol-3-ium chlorure
- 1,4-bis[1-(4-aminophenyl)pyrrolidin-3-yl]-1,4-dimethylpiperazinediium dichlorure
- 1,4-bis[1-(4-aminophenyl)pyrrolidin-3-yl]-1,4-diazoniabicyclo[2.2.2]octane dichlorure
- N,N'-bis{[1-(4-aminophenyl)pyrrolidin-2-yl]methyl}-N,N,N',N'-tetramethylhexane-1,6-diaminium dichlorure
- 3-{[1-(4-amino-3-methylphenyl)pyrrolidin-2-yl]methyl}-1-[6-(1-{[1-(4-amino-3-methylphenyl)pyrrolidin-2-yl]methyl}-1H-imidazol-3-ium-3-yl)hexyl]-1H-imidazol-3-ium dichlorure
- 1,4-bis{[1-(4-aminophenyl)pyrrolidin-2-yl]methyl}-1,4-dimethylpiperazinediium dichlorure
- 1,4-bis{[1-(4-aminophenyl)pyrrolidin-2-yl]methyl}-1,4-diazoniabicyclo[2.2.2]octane dichlorure
- N,N'-bis[1-(4-aminophenyl)pyrrolidin-3-yl]-N,N,N',N'-tetramethylpropane-1,3-diaminium dichlorure
- 3-[1-(4-aminophenyl)pyrrolidin-3-yl]-1-(3-{1-[1-(4-aminophenyl)pyrrolidin-3-yl]-1 H-imidazol-3-ium-3-yl}propyl)-1H-imidazol-3-ium dichlorure
- N,N'-bis{[1-(4-aminophenyl)pyrrolidin-2-yl]methyl}-N,N,N',N'-tetramethylpropane-1,3-diaminium dichlorure
- 3-{[1-(4-aminophenyl)pyrrolidin-2-yl]methyl}-1-[3-(1-{[1-(4-aminophenyl)pyrrolidin-2-yl]methyl}-1H-imidazol-3-ium-3-yl)propyl]-1H-imidazol-3-ium dichlorure
- 1,3-bis(3-{[1-(4-aminophenyl)pyrrolidin-3-yl]amino}propyl)-1H-imidazol-3-ium chlorure
- N,N'-bis[1-(4-aminophenyl)-5-(hydroxymethyl)pyrrolidin-3-yl]-N,N,N',N'-tetramethylhexane-1,6-diaminium dichlorure
- 3-{[1-(4-aminophenyl)-4-hydroxypyrrolidin-2-yl]methyl}-1-[3-(1-{[1-(4-aminophenyl)-4-hydroxypyrrolidin-2-yl]methyl}-1H-imidazol-3-ium-3-yl)propyl]-1H-imidazol-3-ium dichlorure
- 4-[1'-(4-aminophenyl)-2,2'-bipyrrolidin-1-yl]phenylamine
- N,N'-bis[1-(4-aminophenyl)pyrrolidin-5-amido-3-yl]-N,N,N',N'-tetramethylhexane-1,6-diaminium dichlorure
- N,N'-bis[1-(4-aminophenyl)pyrrolidin-3-yl]butane-1,2-diamine
- 1,3-bis{[1-(4-aminophenyl)pyrrolidin-2-yl]methoxy}-propane
- N,N'-bis{[1-(4-aminophenyl)-4-hydroxypyrrolidin-2-yl]methyl}-N,N,N',N'-tetramethylpropane-1,3-diaminium dichlorure
- N,N'-bis[1-(4-aminophenyl)pyrrolidin-3-yl]ethane-1,2-diamine
- N,N'-bis[1-(4-aminophenyl)pyrrolidin-3-yl]-N,N',dipyrrolidinehexane-1,6-diaminium dichlorure.

La composition tinctoriale de la présente invention comprend, dans un milieu cosmétique approprié pour la teinture des fibres kératiniques, en particulier les cheveux humains, à titre de base d'oxydation un dérivé de formule (I) tel que défini précédemment.

La ou les bases d'oxydation de l'invention sont en général présentes chacune en quantité comprise entre 0,001 à 10 % en poids environ du poids total de la composition tinctoriale, de préférence entre 0,005 et 6 %.

La composition tinctoriale de l'invention peut contenir un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les paraphénylènediamines autres que celles décrites précédemment, les bis-phénylalkylènediamines, les para-aminophénols, les bis-paraaminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-à]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-àmino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 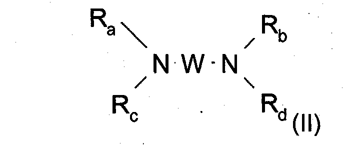 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la couleur désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré. L'agent oxydant peut être l'oxygène de l'air.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du N,N'-Bis-[1-(4-amino-phenyl)-pyrrolidin-3-yl]-N,N'-trimethyl-hexane-1,6-diamonium, chlorure, chlorhydrate (3)

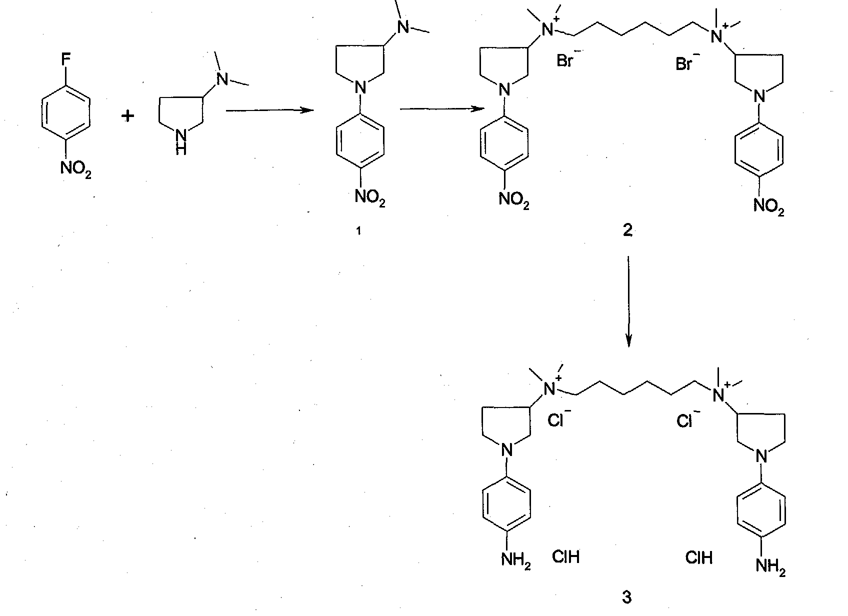

### Synthèse du N,N'-Bis-[1-(4-nitro-phenyl)-pyrrolidin-3-yl]-N,N'-trimethyl-hexane-1,6-diamonium, bromure (2)

0,513g (0,00218 mole) de [1-(4-nitrophenyl)-pyrrolidin-3-yl]-diméthylamine , 0,244 g (0,001 mole) de dibromohexane sont chauffés au reflux dans 10 ml de méthanol pendant six heures. Après retour à la température ambiante, le précipité est lavé à l'acétate d'éthyle puis filtré. Après essorage et séchage du précipité, on obtient 1,5g de poudre jaune (**2**).

RMN 1H (400MHz-DMSO) ppm 8.12(d, 4H) ; 6.75(d, 4H) ; 5.48 (m,2H) ; 3.94(m,2H) ; 3.75(m,4H) ; 3.44(m,7H) ; 3.13(s, 12H) ; 2.51-2.44(m, 4H) ; 1.81(m, 4H) ; 1.39(m,4H). Masse ESI+ : m/z=277 [M2+/2]

### Synthèse du N,N'-Bis-[1-(4-amino-phenyl)-pyrrolidin-3-yl]-N,N'-trimethyl-hexane-1,6-diamonium, chlorure (3)

1,5g (0,0021 mole) du dérivé (2) précédent en solution dans 250ml d'éthanol/ eau (50/50) sont hydrogénés en présence de palladium sur charbon sous une pression d'hydrogène de 10 bars en chauffant à 65°C ; après filtration du catalyseur, le dérivé **(3)** attendu est isolé sous forme de chlorhydrate. On obtient 1g de poudre blanche.

RMN 1H (400MHz-D2O) ppm; 7.20(d, 2H) ; 6.72(d, 2 H); 4.31(m, 2H) ; 3.70(m,2H); 3.55(m, 4H) ; 3.33(m, 4H); 3.21(m, 2H) ; 3.03(bs,12H) ; 2.40(m, 4H) ; 1.79(m,4H) ; 1.37(m, 4H).

Masse ESI+ : m/z=247 [M2+/2]

### Exemple 2 : Synthèse du 3-[1-(4-aminophenyl)pyrrolidin-3-yl]-1-(3-(1-[1-(4-aminophenyl)pyrrolidin-3-yl]-1H-imidazol-3-ium-3-yl}propyl)-1H-imidazol-3-ium dibromide

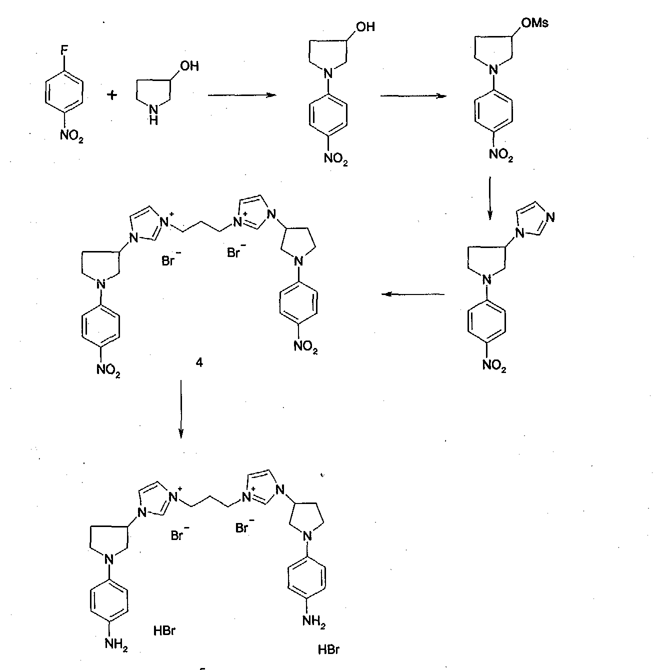

### Synthèse du 3-[1-(4-nitro-phenyl)pyrrolidin-3-yl]-1-(3-{1-[1-(4-nitro-phenyl) pyrrolidin-3-yl]-1H-imidazol-3-ium-3-yl}propyl)-1H-imidazol-3-ium dibromide (4)

3,5g (0,0135 mole) de 1-[1-(4-nitro-phényl)-pyrrolidin-3-yl]-1H-imidazole sont chauffés à 100°C dans 12 ml d'un mélange pentanol / DMF (50/50). 1g (0,005 mole) de dibromopropane sont ajoutés. Le milieu réactionnel est chauffé à 115°C pendant 17 heures. On filtre à chaud , le précipité est lavé au pentanol puis à l'ether. Après essorage et séchage du précipité on obtient 3.4g de poudre jaune (4). Rendement 33%.
RMN 1H (400MHz-DMSO) ppm 9.41(s, 2H) ; 8.13(d, J=9Hz, 4H) ; 7.93(m,2H) ; 7.88(m,2H); 6.71(d, J=9Hz, 4H) ; 5.30(m,2H) ; 4.25(m, 4H) ; 3.98(m,2H) ; 3.83(m, 2H) ; 3.70 (m, 2H) ; 3.60 (m, 2H) ; 2.46-2.40(m, 6H).
Masse ESI+ : m/z=639 [M²⁺ + Br⁻]⁺, 557 [M²⁺ + Br⁻ - HBr]⁺

### Synthèse du 3-[1-(4-aminophenyl)pyrrolidin-3-yl]-1-(3-{1-[1-(4-aminophenyl) pyrrolidin-3-yl]-1H-imidazol-3-ium-3-yl}propyl)-1H-imidazol-3-ium dibromure (5)

3,2g (0,0044 mole) du dérivé (4) précédent en solution dans un mélange 60ml d'eau /300ml d'éthanol sont hydrogénés en présence de palladium sur charbon sous une pression d'hydrogène de 9 bars et à 75°C. Après filtration du catalyseur, le dérivé **(5)** attendu est isolé sous forme de bromhydrate. On obtient 3,4g de poudre blanche, rendement 94%.

RMN 1H (400MHz-DMSO) ppm 8.84 (s, 2H) ; 7.41 (s, 2H) ; 7.41 (s, 2H) ; 7.17 (d, 4H) ; 6.69 (d, 4H) ; 5.14 (m, 2H) ; 4.22 (t, 4H) ; 3.69 (dd, 2H) ; 3.62 (dd, 2H) ; 3.55 (m, 2H) ; 3.34 (m, 2H) ; 2.59 (m, 2H) ; 2.42 (m, 2H) ; 2.3 (m, 2H).
Masse ESI+ : m/z=579 [M²⁺ + Br⁻]⁺, 249 [M]²⁺ /2

### Exemple 3 : Synthèse du 1,1'Bis-(4-Amino-phenyl)-[2,2']bipyrrolidininyl-1-yl

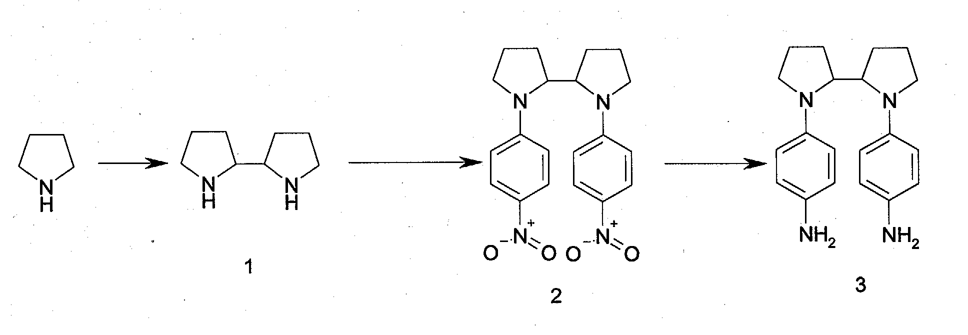

### Préparation du [2,2'] Bipyrrolidinyl (1)

108 g (1,52 mol) de pyrrolidine et 11 g (0,076 mol) de di-t-butylperoxide sont chauffées dans un autoclave inox à 140°C pendant 9 heures. Le milieu réactionnel est évaporé à sec puis traité avec le borohydrure de sodium dans l'éthanol pendant 1 heure. Après évaporation de l'éthanol, le produit est extrait à l'acétate d'éthyle et lavé à l'eau. En concentrant, on obtient 2.6 g (80%) d'une huile brune engagée telle quelle pour la suite de la synthèse.

### Préparation du 1,1'-Bis-(4-nitro-phenyl)-[2,2'] Bipyrrolidinyl (2)

2,6 g (∼15 mmol) du [2,2'] Bipyrrolidinyl (1) obtenu ci-dessus est porté à reflux en présence de carbonate de potassium 5g (35,7 mmol) et de para-fluoronitrobenzène 5g (35,7 mmol) dans 25 ml d'eau pendant 1 nuit. L'agglomérat formé est filtré puis rincé à l'acétone et l'isopropanol pour donner 2,15g d'un mélange de deux isomères (55/45) (44%). Après purification chromatographique sur gel de silice, on isole un isomère (1,2g) majoritaire dont les analyses sont :
^{**1**}**H RMN (400MHz, DMSO)** : 1,92 (m, 2H, CH₂ 3') ; 1,98 (m, 2H, CH₂ 4') ; 2,11 (m, 2H, CH₂ 4') ; 2,48 (m, 2H, CH₂ 3' ) ; 3,14 (m, 2H, 1H, CH₂ 2') ; 3,55 (m, 2H, CH₂ 2') ; 4,13 (m, 2H, CH 5') ; 6,39 (d, 4H, H2-H6) ; 7,81 (d, 4H, H3-H5).
Masse ESI+ : m/z = 383 (M+H)⁺

### Préparation du 1,1'-Bis-(4-amino-phenyl)-[2,2'] Bipyrrolidinyl (3)

Le dérivé (2) est mis en solution dans 300ml d'éthanol puis hydrogéné en présence de zinc. Le dérivé (3) attendu est isolé sous forme de chlorhydrate après avoir acidifié le filtrat avec de l'acide chlorhydrique.

### EXEMPLES 1 A 4 DE TEINTURE EN MILIEU ALCALIN

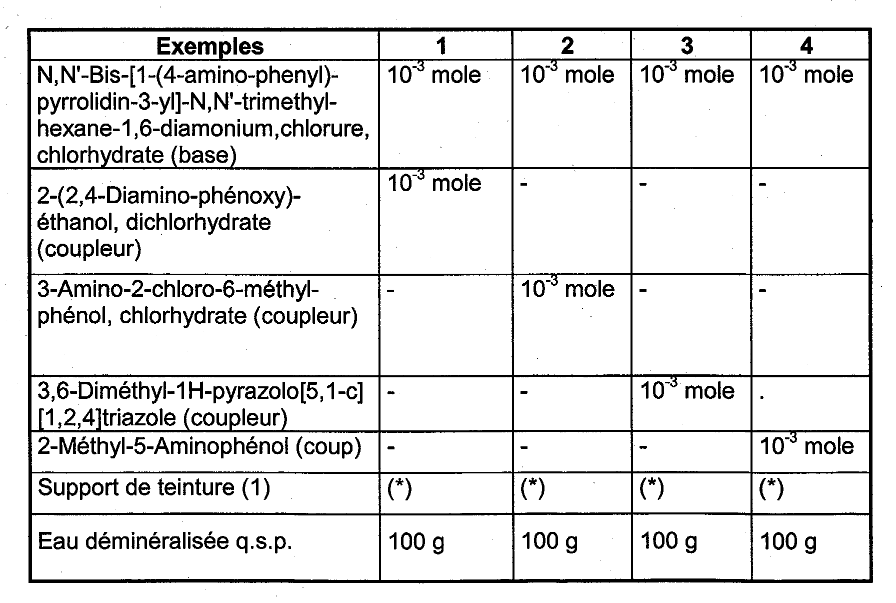

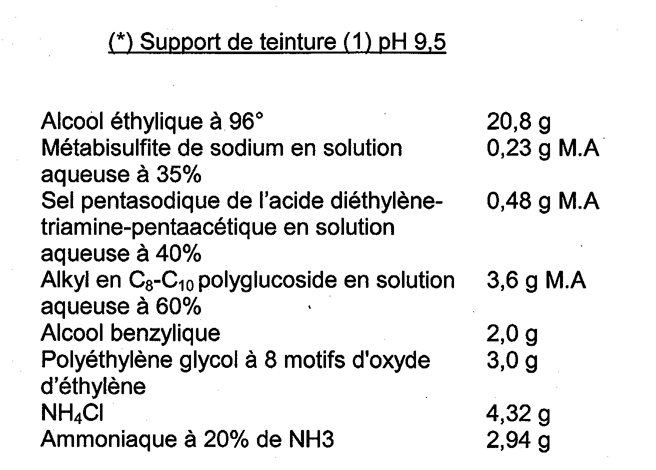

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

| **Exemples** | **1** | **2** | **3** | **4** |
|---|---|---|---|---|
| **Nuance observée** | Bleu violet | Violet bleu | Violet rouge | Violet |

### EXEMPLES 5 A 7 DE TEINTURE EN MILIEU ACIDE

On a préparé les compositions tinctoriales suivantes :

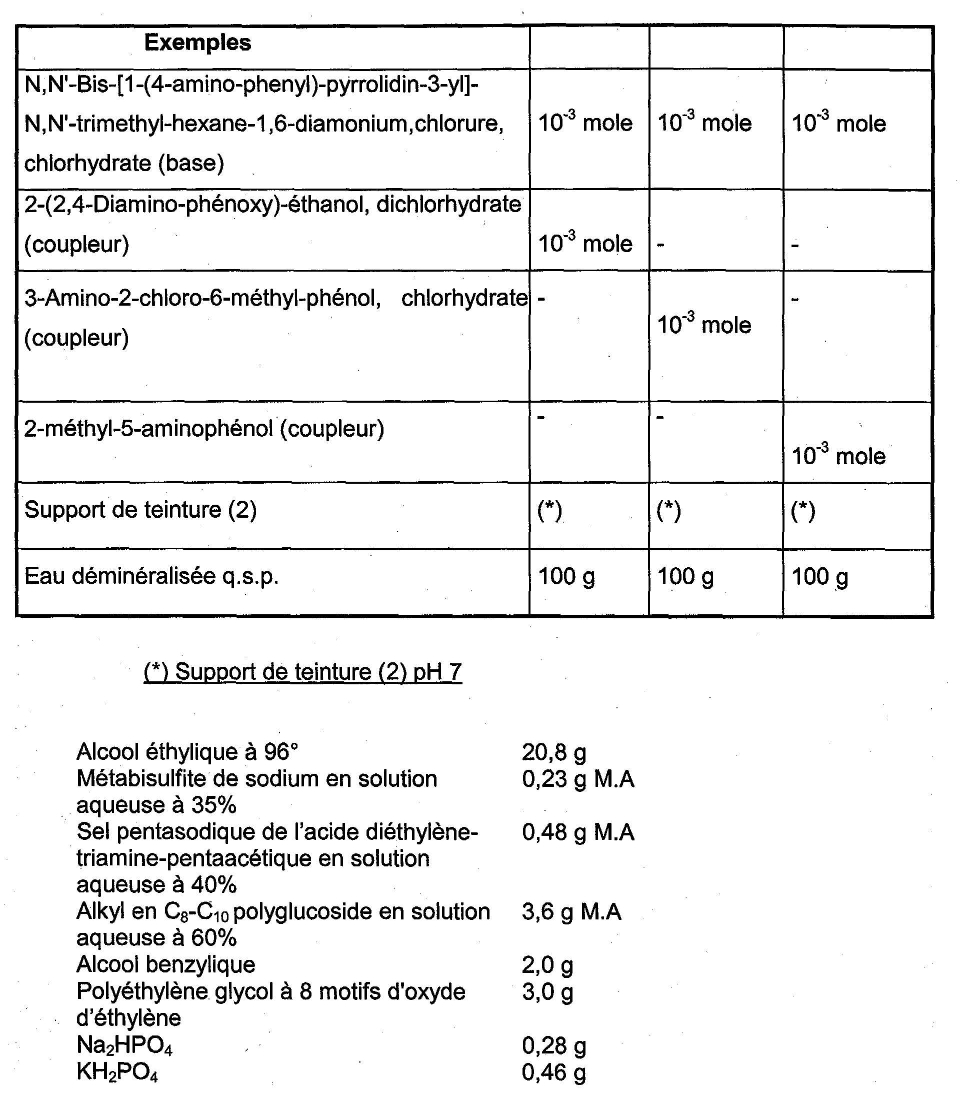

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

| **Exemples** | **5** | **6** | **7** |
|---|---|---|---|
| **Nuance observée** | Bleu violet | Violet bleu | violet |

## Revendications

1. Dérivés de bis-para-phénylènediamine substitués par un groupement pyrrolidinyle de formule (I) et leurs sels d'addition 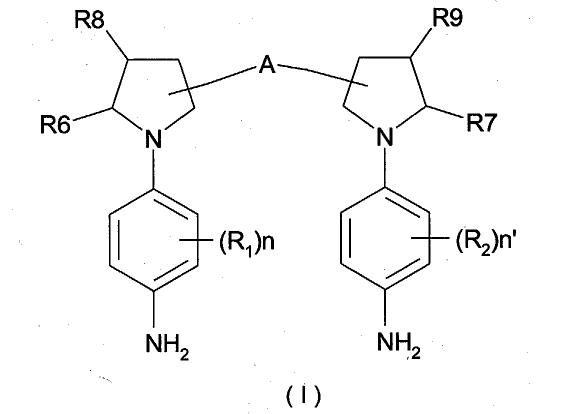 dans laquelle :
• n et n', indépendamment l'un de l'autre sont compris entre 0 et 4 inclus, étant entendu que lorsque n, respectivement n' est supérieur ou égal à 2 alors les radicaux R₁ et R₂ peuvent être identiques ou différents,
• R₁ et R₂ représentent indépendamment un atome d'halogène ; une chaîne hydrocarbonée en C₁-C₆, aliphatique ou alicyclique, saturée ou insaturée, un ou plusieurs des atomes de carbone de la chaîne pouvant être remplacés par un atome d'oxygène, d'azote, de silicium, de soufre, par un groupement SO ou par un groupement SO₂, les radicaux R₁ et R₂ ne comportant pas de liaison peroxyde, ni de radicaux diazo, nitro ou nitroso , la chaîne pouvant être substituée par un ou plusieurs atomes d'halogène, un ou plusieurs radicaux hydroxy, alcoxy en C₁-C₆, amino, mono- ou di- alkyl(C₁-C₆)amino, trialkyl(C₁-C₆)ammonium, un radical N-alkyl(C₁-C₆)imidazolinium
• A est une liaison covalente ou une chaîne alkylène comportant de 1 à 14 atomes de carbone, linéaire ou ramifiée, saturée ou insaturée, un ou plusieurs des atomes de carbone de la chaîne pouvant être remplacés par un radical onium Z et/ou par un atome choisi parmi les atomes d'oxygène, de soufre, de silicium ou d'azote, un groupe CO, SO ou SO₂, la chaîne pouvant être substituée par un ou plusieurs atomes d'halogène, un ou plusieurs radicaux hydroxyle, alcoxy en C₁-C₆, amino, alkyl(C₁-C₆)amino ou di-alkyl(C₁-C₆)amino ;
• R₆ et R₇ représentent indépendamment un atome d'hydrogène ; un radical carboxyle ; un radical alkyl(C₁-C₄)carboxyle ; un radical carbamoyle ; un radical (alkyl ou dialkyl)(C₁-C₄) carbamoyle ; un radical trialkyl(C₁-C₆)silane, un radical tri-alkyl(C₁-C₆)ammonium ; un radical N-alkyl(C₁-C₆)imidazolinium ; un radical alkyle en C₁-C₁₅ ; un radical alkyle en C₁-C₁₅ pouvant être insaturé, substitué par un ou plusieurs radicaux hydroxy, alkyl(C₁-C₆)oxy, amino, mono- ou di-alkyl(C₁-C₆)amino, thiol, alkyl(C₁-C₆)sulfonique ou halogène ; un radical alkyle en C₁-C₁₅ pouvant être insaturé, substitué par un ou plusieurs radicaux carboxylique, alkyl (C₁-C₆)carbonyle, alcoxy (C₁-C₆)carbonyle, carbamoyle, mono- ou di-alkyl (C₁-C₆)carbamoyle, trialkyl(C₁-C₆)silane, tri-alkyl(C₁-C₆)ammonium ou N-alkyl(C₁-C₆)imidazolinium.
• R₈ et R₉ représentent indépendamment un atome d'hydrogène ; un radical hydroxyle ; un radical alkyl(C₁-C₄)oxy, un radical amino ; un radical mono- ou di-alkyl(C₁-C₄)amino ; un radical thiol ; un radical carboxyle ; un radical alkyl(C₁-C₄)carboxyle ; un radical carbamoyle ; un radical (alkyl ou dialkyl)(C₁-C₄) carbamoyle ; un radical trialkyl(C₁-C₆)silane, un radical tri(alkyl(C₁-C₆)ammonium ; un radical N-alkyl(C₁-C₆)imidazolinium ; un radical alkyle en C₁-C₁₅ ; un radical alkyle en C₁-C₁₅ pouvant être insaturé, substitué par un ou plusieurs radicaux hydroxy, alkyl(C₁-C₆)oxy, amino, mono- ou di-alkyl(C₁-C₆)amino, thiol, alkyl(C₁-C₆)sulfonique ou halogène ; un radical alkyle en C₁-C₁₅ pouvant être insaturé, substitué par un ou plusieurs radicaux carboxylique, alkyl (C₁-C₆)carbonyle, alcoxy (C₁-C₆)carbonyle, carbamoyle, mono- ou di-alkyl (C₁-C₆)carbamoyle , trialkyl(C₁-C₆)silane, tri(alkyl(C₁-C₆)ammonium , ouN-alkyl(C₁-C₆)imidazolinium.

2. Dérivés selon la revendication 1 dans lesquels n et n' sont indépendamment 0 ou 1.

3. Dérivés selon la revendication 1 ou 2 dans lesquels R₁ et R₂ sont indépendamment choisis parmi le chlore, le brome, un radical alkyle en C₁-C₄, un radical hydroxyalkyle en C₁-C₄, un radical aminoalkyle en C₁-C₄, un radical alcoxy en C₁-C₄, un radical hydroxyalcoxy en C₁-C₄, un radical trialkyl(C₁-C₄)ammonium-alkyle en C₁-C₄, un radical N-alkyl(C1-C4)imidazoliniumalkyle en C1-C4.

4. Dérivés selon la revendication 3 dans lesquels R₁ et R₂ sont choisis parmi un radical méthyle, isopropyle, terbutyle, hydroxyméthyle, 2-hydroxyéthyle, 1,2-dihydroxyéthyle, méthoxy, isopropyloxy, 2-hydroxyéthoxy, triméthylammoniumméthyle, N-méthylimidazolinium.

5. Dérivés selon l'une quelconque des revendications 1 à 4 dans lesquels A ne comporte pas de liaison peroxyde, ni de radicaux diazo, nitro ou nitroso.

6. Dérivés selon l'une quelconque des revendications 1 à 5 dans lesquels A est une liaison covalente ou une chaîne alkylène comportant de 1 à 8 atomes de carbone.

7. Dérivés selon l'une quelconque des revendications 1 à 5 dans lesquels A est une chaîne alkylène comprenant de 1 à 8 atomes de carbone, un ou plusieurs des atomes de carbone de la chaîne pouvant être remplacés par un atome d'azote et/ou un atome d'oxygène.

8. Dérivés selon l'une quelconque des revendications 1 à 5 dans lesquels A est une chaîne alkylène, dont un ou plusieurs atomes de carbone de la chaîne peuvent être remplacés par un atome d'azote et/ou d'oxygène, et comportant un ou plusieurs radicaux onium Z.

9. Dérivés selon l'une quelconque des revendications 1 à 8 dans lesquels le radical onium Z correspond à la formule (II) : 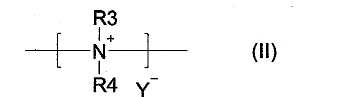 dans laquelle
• R₃ et R₄, pris séparément, représentent un radical alkyle en C₁-C₁₅ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical amidoalkyle en C₁-C₆ ; un radical trialkyl(C₁-C₆)silanealkyle en C₁-C₆ ; un radical aminoalkyle en C₁-C₆ ; un radical aminoalkyle en C₁-C₆ dont l'amine est mono- ou di-substituée par un radical alkyle en C₁-C₄, alkyl(C₁-C₆)carbonyle, amido ou alkyl(C₁-C₆)sulfonyle ;
• R₃ et R₄ ensemble, forment, avec l'atome d'azote auxquels ils sont rattachés, un hétérocycle à 5, 6 ou 7 chaînons ;
• lorsqu'au moins 2 radicaux oniums de formule (II) sont présents dans la chaîne A, les radicaux R3 et/ou R4 d'un des radicaux onium peuvent former avec les radicaux R3 et/ou R4 de l'autre radical onium une structure cyclique diammonium,
• Y⁻ est un contre ion.

10. Dérivés selon la revendication 9 dans lesquels R₃ et R₄, pris séparément sont choisis parmi les radicaux alkyles en C₁-C₄, les radicaux hydroxyalkyles en C₁-C₄.

11. Dérivés selon la revendication 9 dans lesquels R₃ et R₄ ensemble forment un cycle cationique pyrrolidinium, pipéridinium, pipérazinium ou morpholinium, le cycle cationique pouvant être substitué par un atome d'halogène, un radical hydroxyle, alkyle en C₁-C₆, monohydroxyalkyle en C₁-C₆, polyhydroxyalkyle en C₂-C₆, alcoxy en C₁-C₆, trialkyl(C₁-C₆)silanealkyle en C₁-C₆, amido, carboxyle, alkyle(C₁-C₆), thio, thioalkyle en C₁-C₆, alkyl(C₁-C₆)thio, amino, amino mono ou disubstitué par un radical alkyl(C₁-C₆), alkyl(C₁-C₆)carbonyle, amido ou alkyl(C₁-C₆)sulfonyle.

12. Dérivés selon la revendication 11 dans lesquels R₃ et R₄ ensemble forment un cycle cationique pyrrolidinium, pipéridinium et morpholinium.

13. Dérivés selon l'une quelconque des revendications précédentes 1 à 8 dans lesquels le radical onium Z correspond à la formule (III) : 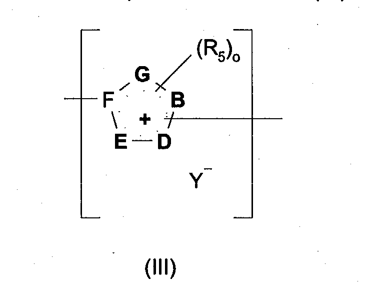 dans laquelle
• les sommets B, D, E, F, G identiques ou différents, représentent un atome de carbone, d'oxygène, de soufre ou d'azote nécessaire pour former un cycle aromatique cationisé sur l'azote de type pyrolium, pyrazolium, imidazolium, triazolium, oxazolium, isooxazolium, thiazolium, isothiazolium ; o est un nombre entier compris entre 0 et 4 inclus ;
• R₅, identique ou différent, représente un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; lorsque les radicaux R₅ sont portés par un carbone, ils représentent également un radical hydroxyle, alkyl(C₁-C₄)oxy, amino, alkyl(C₁-C₄)amino ou di alkyl(C₁-C₄)amino, et
• Y⁻ est un contre-ion.

14. Dérivés selon la revendication 13 dans lesquels le cycle aromatique cationique est choisi parmi le cycle imidazolium et le cycle thiazolium.

15. Dérivés selon l'une quelconque des revendications précédentes 1 à 8 dans lesquels le radical onium Z correspond à la formule (IV) : 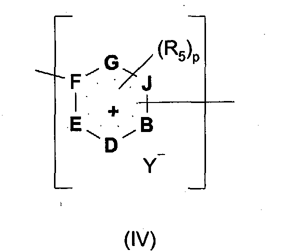 dans laquelle :
• les sommets B, D, E, F, G et J, identiques ou différents, représentent un atome de carbone ou d'azote pour former un cycle aromatique cationisé sur l'azote de type pyridinium, pyrimidinium, pyrazinium, triazinium et pyridazinium ;
• p est un nombre entier compris entre 0 et 4 inclus ;
• R₅, identique ou différent, représente un radical alkyle en C₁-C₆, un radical monohydroxyalkyle en C₁-C₆, un radical polyhydroxyalkyle en C₂-C₆, un radical trialkyl(C₁-C₆)silanealkyle en C₁-C₆, un radical alcoxy(C₁-C₆)alkyle en C₁-C₆, un radical carbamylalkyle C₁-C₆, un radical alkyl(C₁-C₆)carboxyalkyle en C₁-C₆, un radical benzyle ; lorsque les radicaux R₅ sont portés par un carbone, ils représentent également un radical hydroxyle, alkyl(C₁-C₄)oxy, alkyl(C₁-C₄)amino ou di alkyl(C₁-C₄)amino, et
• Y⁻ représente un contre ion.

16. Dérivés selon la revendication 15 dans lesquels le radical onium Z de formule (IV) est un cycle cationisé pyridinium.

17. Dérivés selon l'une quelconque des revendications 13 à 16 dans lesquels R₅ est un radical alkyle en C₁-C₄ ou hydroxyalkyle en C₁-C₄.

18. Dérivés selon l'une quelconque des revendications précédentes dans lesquels R₆ et R₇ sont choisis parmi l'hydrogène, un radical hydroxyalkyle en C₁-C₄, un radical alkyle en C₁-C₄, un radical carboxyle, un radical carbamoyle, un radical mono ou dialkyl(C₁-C₄)) carbamoyle, un radical trialkyl(C₁-C₄)ammoniumalkyl en C₁-C₄, un radical N-alkyl(C₁-C₄)imidazoliumalkyle en C₁-C₄.

19. Dérivés selon l'une quelconque des revendications précédentes dans lesquels R₈ et R₉ sont choisis parmi l'hydrogène, un radical hydroxyle, un radical amino, un radical mono ou dialkyl (C₁-C₄)amino, un radical alkyle en C₁-C₄, un radical trialkyl(C₁-C₄)ammonium, un radical N-alkyl(C₁-C₄)imidazolinium.

20. Dérivés selon l'une quelconque des revendications précédentes de formule (I') 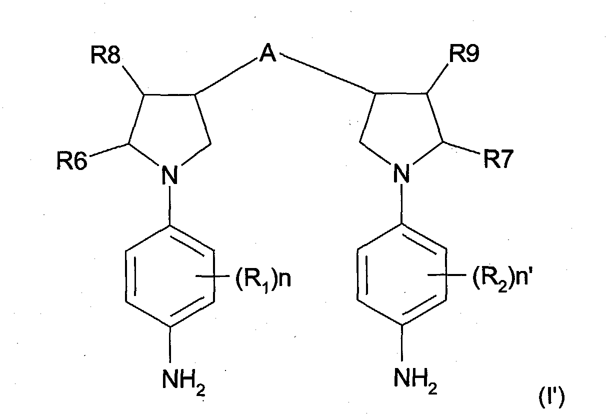 dans lesquels R₁, R₂, n, n', R₆, R₇, R₈, R₉ et A sont tels que définis précédemment.

21. Dérivés selon la revendication 20 dans lesquels n et n' sont 0 ou 1, R₁ et R₂ sont choisis parmi un radical méthyle, isopropyle, terbutyle, hydroxyméthyle, 2-hydroxyéthyle, 1,2-dihydroxyéthyle, méthoxy, isopropoxy, 2-hydroxyéthoxy, triméthylammoniumméthyle, un radical N-méthylmidazoliniumméthyle ,R₆ et R₇ sont choisis parmi un atome d'hydrogène, un radical hydroxyalkyle en C₁-C₄, un radical alkyle en C₁-C₄, un radical carboxyle, un radical carbamoyle, un radical mono ou dialkyl(C₁-C₄)carbamoyle, un radical trialkyl(C₁-C₄)ammoniumalkyl een C₁-C₄ , un radical N-alkyl(C₁-C₄)imidazoliumalkyle, R₈ et R₉ sont choisis parmi un atome d'hydrogène, un radical hydroxyle, un radical amino, un radical mono ou dialkyl (C₁-C₄))amino, un radical alkyle en C₁-C₄, un radical trialkyl(C₁-C₄)ammonium, un radical N-alkyl(C₁-C₄)imidazolinium, A est une liaison covalente ou un radical choisi parmi :

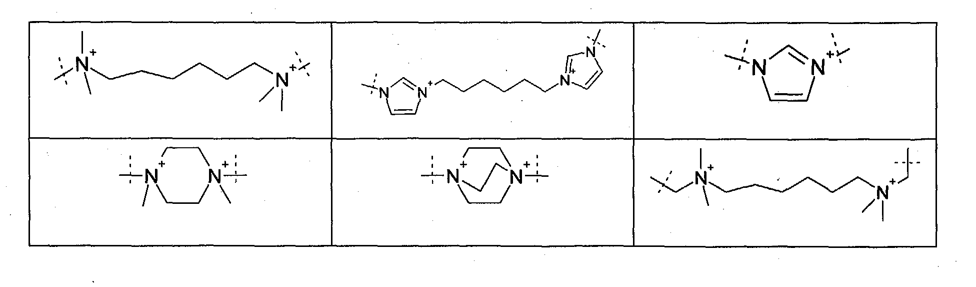

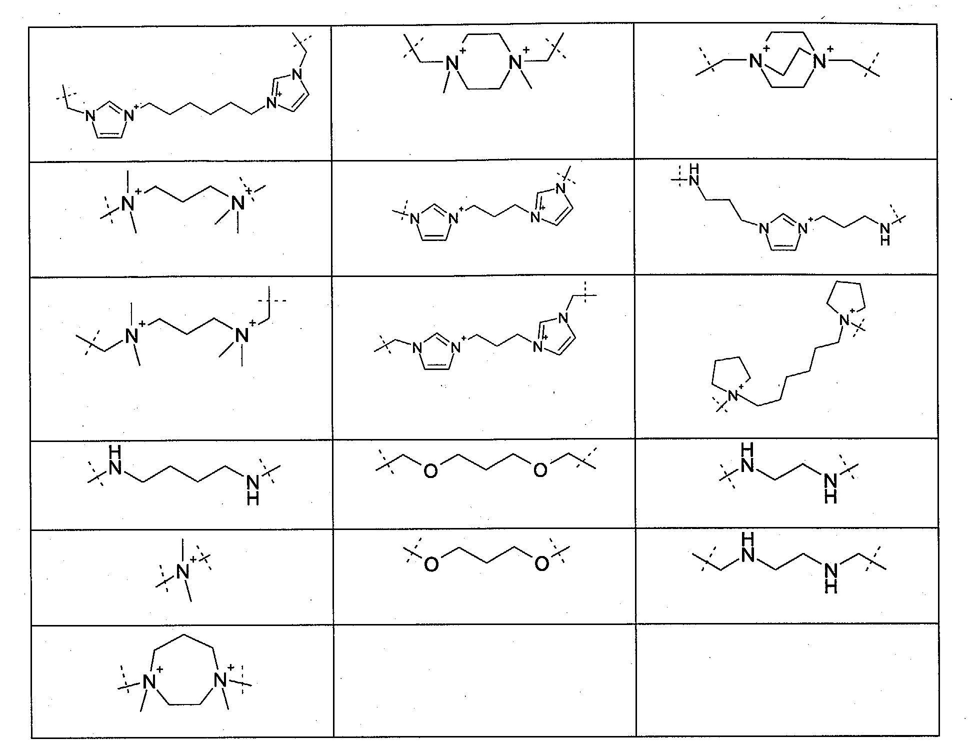

22. Dérivés selon l'une quelconque des revendications 1 à 21 choisis parmi
- N,N'-bis[1-(4-aminophenyl)pyrrolidin-3-yl]-N,N,N',N'-tetramethylhexane-1,6-diaminium dichlorure
- 3-[1-(4-aminophenyl)pyrrolidin-3-yl]-1-(6-{1-[1-(4-aminophenyl)pyrrolidin-3-yl]-1H-imidazol-3-ium-3-yl}hexyl)-1H-imidazol-3-ium dichlorure
- 1,3-bis[1-(4-aminophenyl)pyrrolidin-3-yl]-1H-imidazol-3-ium chlorure
- 1,4-bis[1-(4-aminophenyl)pyrrolidin-3-yl]-1,4-dimethylpiperazinediium dichlorure
- 1,4-bis[1-(4-aminophenyl)pyrrolidin-3-yl]-1,4-diazoniabicyclo[2.2.2]octane dichlorure
- N,N'-bis{[1-(4-aminophenyl)pyrrolidin-2-yl]methyl}-N,N,N',N'-tetramethylhexane-1,6-diaminium dichlorure
- 3-{[1-(4-amino-3-methylphenyl)pyrrolidin-2-yl]methyl}-1-[6-(1-{[1-(4-amino-3-methylphenyl)pyrrolidin-2-yl]methyl}-1H-imidazol-3-ium-3-yl)hexyl]-1H-imidazol-3-ium dichlorure
- 1,4-bis{[1-(4-aminophenyl)pyrrolidin-2-yl]methyl}-1,4-dimethylpiperazinediium dichlorure
- 1,4-bis{[1-(4-aminophenyl)pyrrolidin-2-yl]methyl}-1,4-diazoniabicyclo[2.2.2]octane dichlorure
- N,N'-bis[1-(4-aminophenyl)pyrrolidin-3-yl]-N,N,N',N'-tetramethylpropane-1,3-diaminium dichlorure
- 3-[1-(4-aminophenyl)pyrrolidin-3-yl]-1-(3-{1-[1-(4-aminophenyl)pyrrolidin-3-yl]-1H-imidazol-3-ium-3-yl}propyl)-1H-imidazol-3-ium dichlorure
- N,N'-bis{[1-(4-aminophenyl)pyrrolidin-2-yl]methyl}-N,N,N',N'-tetramethylpropane-1,3-diaminium dichlorure
- 3-{[1-(4-aminophenyl)pyrrolidin-2-yl]methyl}-1-[3-(1-{[1-(4-aminophenyl)pyrrolidin-2-yl]methyl}-1H-imidazol-3-ium-3-yl)propyl]-1H-imidazol-3-ium dichlorure
- 1,3-bis(3-{[1-(4-aminophenyl)pyrrolidin-3-yl]amino}propyl)-1H-imidazol-3-ium chlorure
- N,N'-bis[1-(4-aminophenyl)-5-(hydroxymethyl)pyrrolidin-3-yl]-N,N,N',N'-tetramethylhexane-1,6-diaminium dichlorure
- 3-{[1-(4-aminophenyl)-4-hydroxypyrrolidin-2-yl]methyl}-1-[3-(1-{[1-(4-aminophenyl)-4-hydroxypyrrolidin-2-yl]methyl}-1H-imidazol-3-ium-3-yl)propyl]-1H-imidazol-3-ium dichlorure
- 4-[1'-(4-aminophenyl)-2,2'-bipyrrolidin-1-yl]phenylamine
- N,N'-bis[1-(4-aminophenyl)pyrrolidin-5-amido-3-yl]-N,N,N',N'-tetramethylhexane-1,6-diaminium dichlorure
- N,N'-bis[1-(4-aminophenyl)pyrrolidin-3-yl]butane-1,2-diamine
- 1,3-bis{[1-(4-aminophenyl)pyrrolidin-2-yl]methoxy}-propane
- N,N'-bis{(1-(4-aminophenyl)-4-hydroxypyrrolidin-2-yl]methyl}-N,N,N',N'-tetramethylpropane-1,3-diaminium dichlorure
- N,N'-bis[1-(4-aminophenyl)pyrrolidin-3-yl]ethane-1,2-diamine
- N,N'-bis[1-(4-aminophenyl)pyrrolidin-3-yl]-N,N',dipyrrolidinehexane-1,6-diaminium dichlorure

23. Composition tinctoriale comprenant à titre de base d'oxydation au moins un dérivé paraphénylènediamine de formule (I), tel que défini selon l'une quelconque des revendications 1 à 22.

24. Composition selon la revendication 23 comprenant de plus un coupleur choisi parmi les métaphénylènediamines, les méta-aminophénols, les métadiphénols, les coupleurs naphtaléniques, les coupleurs hétérocycliques et leurs sels d'addition

25. Composition selon l'une quelconque des revendications 23 ou 24 comprenant une base d'oxydation additionnelle autre que les bases d'oxydation de formule (I) choisie parmi les paraphénylènediamines, les bis-phénylalkylènediamines, les para-aminophénols, les ortho-aminophénols, les bases hétérocycliques et leurs sels d'addition.

26. Composition selon l'une quelconque des revendications 23 à 25 dans laquelle la quantité de chacune des bases d'oxydation est comprise entre 0,001 à 10 % en poids environ du poids total de la composition tinctoriale.

27. Composition selon l'une quelconque des revendications 24 à 26 dans laquelle la quantité de chacun des coupleurs est comprise entre 0,001 et 10 % en poids environ du poids total de la composition tinctoriale.

28. Composition selon l'une quelconque des revendications 23 à 27 comprenant de plus un milieu cosmétique approprié à la teinture des fibres . kératiniques.

29. Procédé de teinture d'oxydation des fibres kératiniques, **caractérisé en ce qu'**on applique sur les fibres une composition tinctoriale telle que définie dans l'une quelconque des revendications 23 à 28 en présence d'un agent oxydant pendant un temps suffisant pour développer la coloration désirée.

30. Procédé selon la revendication 29 dans lequel l'agent oxydant est choisi parmi le peroxyde d'hydrogène, le peroxyde d'urée, les bromates de métaux alcalins, les persels, les peracides et les enzymes oxydases.

31. Dispositif à plusieurs compartiments dans lequel un premier compartiment contient une composition tinctoriale telle que définie à l'une quelconque des revendications 23 à 28 et un deuxième compartiment contient un agent oxydant.

32. Utilisation de la composition définie à l'une quelconque des revendications 23 à 28 pour la teinture de fibres kératiniques.
